# EUROPEAN PATENT APPLICATION

(11) **EP 3 482 682 A1**
(43) Date of publication of application: **15.05.2019**
(21) Application number: 17200510.0
(22) Date of filing: 08.11.2017
(51) Int. Cl.: A61B 5/021, A61B 5/022, A61B 5/00, A61B 5/0225

(54) **BLOOD PRESSURE MEASUREMENT DEVICE**

(71) Applicant: Koninklijke Philips N.V., 5656 AE Eindhoven (NL)
(72) Inventor: HILBIG, Rainer, 5656 AE Eindhoven (NL); HILGERS, Achim, 5656 AE Eindhoven (NL); VAN DER SLUIS, Paul, 5656 AE Eindhoven (NL)
(74) Representative: de Haan, Poul Erik

(57) **Abstract**

A blood pressure measurement device (10) is disclosed comprising a cuff (20) having an inflatable compartment (21) in fluid communication with a chamber (30) comprising an electrode arrangement (33, 33') contacting at least a first matter (31, 32) capable of releasing a gas having a composition different to said matter for inflating the inflatable compartment in response to an electromagnetic stimulus applied to the at least the first matter by the electrode arrangement; and a power supply unit (40) including a controller (41) communicatively coupled to the electrode arrangement adapted to control the power supply unit to provide said electromagnetic stimulus to the electrode arrangement.

## Description

### FIELD OF THE INVENTION

The present invention relates to a blood pressure measurement device comprising a cuff having an inflatable compartment and a power supply unit including a controller adapted to control the inflation of the inflatable compartment.

### BACKGROUND OF THE INVENTION

Vital signs are measurements of the human body's most basic functions. Commonly monitored vital signs include blood pressure and heart rate (pulse rate), which may be used to diagnose a range of medical conditions such as hypertension, hypotension, bradycardia, tachycardia, and so on. Also, the heart rhythm and strength of the pulse determined during such vital signs monitoring may provide important clinical information.

The monitoring of blood pressure and pulse rate may be performed using a number of different techniques. For instance, blood pressure is typically measured with a blood pressure cuff (sphygmomanometer) and either manually with a stethoscope (auscultatory method) by listening to Korotkoff's sounds or automatically (electronically) by detecting changes of oscillations (oscillometric method) in the blood vessels. The blood pressure may be measured by a medical professional such as a general practitioner, nurse or other health care provider in a medical environment or at home by the patient/user typically itself, where usually oscillometry-based approaches are used.

An example of a blood pressure measurement device for home use using such an oscillometric method is disclosed in US2017/224229 A1. The blood pressure measurement device disclosed herein is equipped with a pressing surface having plural pressure sensors arranged in one direction, an air bag for pressing the pressing surface against a living body part in a state that the one direction crosses a direction in which the radius artery T runs, an air bag drive unit, a rotational drive unit for driving the pressing surface rotationally about at least one of axes X and Y, and a control unit which performs a rotation control on the basis of pressure pulse waves that were detected by the pressure sensors in a process that the pressing force was increased and calculates blood pressure values on the basis of pressure pulse waves that were detected by the pressure sensors after the rotation control in a process that the pressing force was decreased. Several other types of blood pressure measurement devices are disclosed in "A review of methods for non-invasive and continuous blood pressure monitoring: Pulse transit time method is promising?" by P.L Nouty et al. in IRBM, Volume 35, Issue 5, October 2014, pages 271-282.

Existing blood pressure measurement devices based on the oscillometric method have in common that they require an air pump or similar mechanical device to inflate the cuff surrounding part of a patient's limb, e.g. upper arm, wrist or finger, such that the blood pressure can be measured. This has a number of drawbacks. Firstly, such mechanical devices are noisy during their operation, which can be perceived as being unpleasant or disturbing. Moreover, such mechanical devices typically must be housed in a relatively bulky control unit, which is not ideal if the blood pressure measurement device is to be used for (semi-)continuous blood pressure monitoring over a prolonged period of time, during which the patient has to wear the blood pressure measurement device.

### SUMMARY OF THE INVENTION

The present invention seeks to provide a blood pressure measurement device according to the opening paragraph in which at least some of the drawbacks of existing blood pressure measurement devices have been overcome.

According to an aspect, there is provided a blood pressure measurement device comprising a cuff having an inflatable compartment in fluid communication with a chamber comprising an electrode arrangement contacting at least a first matter capable of releasing a gas having a composition different to said matter for inflating the inflatable compartment in response to an electromagnetic stimulus applied to the at least the first matter by the electrode arrangement; and a power supply unit including a controller communicatively coupled to the electrode arrangement adapted to control the power supply unit to provide said electromagnetic stimulus to the electrode arrangement.

The present invention is based on the insight that different types of matter, e.g. materials or substances, exist that upon being exposed to an electromagnetic stimulus such as heat or an electric current can release gas that is stored or otherwise locked into the matter, e.g. through adsorption or chemical binding. Consequently, by controlling the release of the gas (or multiple gases) from such a matter with the controller by controlled application of the electromagnetic stimulus to the matter, the inflatable compartment of the cuff may be inflated without requiring mechanically moving parts such as an air pump or the like, thereby providing a blood pressure measurement device that can be silently operated and it can be made more compact than the aforementioned prior art blood pressure measurement devices.

The blood pressure measurement device may further comprise a pressure sensor in said cuff communicatively coupled to the controller, wherein the controller is adapted to control the power supply unit to provide said electromagnetic stimulus to the electrode arrangement as a function of a pressure in the inflatable compartment sensed by the pressure sensor. Such a feedback mechanism ensures that the pressure within the cuff can be accurately controlled, and more importantly, ensures that the generation of the electromagnetic stimulus can be tailored to the generation of the appropriate amounts of gas from the at least one matter in the chamber. For example, the controller may be adapted to use the pressure feedback information from the pressure sensor to maintain a baseline pressure within the cuff, whilst periodically increasing the baseline pressure to perform a blood pressure measurement. This is particularly advantageous where the cuff is to be worn over a longer period of time by a patient, e.g. to facilitate periodic blood pressure measurements, such that the baseline pressure ensures a comfortable yet secure fit of the cuff around a portion of a limb of the patient, such as the patient's bicep, wrist or finger. Alternatively or additionally, the blood pressure measurement device may further comprise a valve in fluid communication with the inflatable compartment, wherein the controller is arranged to control said valve to regulate inflation and/or deflation of the inflatable compartment in order to control the pressure in the cuff.

In an embodiment, the chamber and the power supply unit are housed in a control unit in fluid communication with the inflatable compartment through a length of tubing such that the volume of the cuff can be minimalized. Alternatively, the chamber is housed in a control unit mounted on the cuff such that no separate control unit is required thereby making the blood pressure measurement device more portable, which for example is advantageous where the blood pressure measurement device is to be worn by a patient whilst that the patient is on the move. In yet another embodiment, the cuff comprises a further compartment housing the chamber in fluid communication with the inflatable compartment. The further compartment may further include the power supply unit in order to provide a blood pressure measurement device that is particularly compact and portable. For example, in this embodiment the cuff may be dimensioned to fit around a finger to yield a blood pressure measurement device that provides minimal discomfort to its wearer although it should be understood that the blood pressure measurement device according to embodiments of the present invention may be worn around any suitable body part such as the upper arm (bicep) or wrist of the patient.

In a first set of embodiments, the first matter comprises a zeolite material and wherein the electrode arrangement comprises a heating element adapted to heat the zeolite material. This set of embodiments is based on the realization that zeolite materials are available that have the ability to absorb gases, with the gas absorption capacity of such materials being temperature-dependent. Consequently, by controlling the temperature of such a zeolite material, the amount of gas stored in the zeolite material can be controlled, e.g. reduced to inflate the cuff or increased to deflate the cuff, without requiring moving parts, thus resulting in a blood pressure measurement device that can be operated silently.

In this set of embodiments, the blood pressure measurement device may further comprise a cooling element in thermal contact with the zeolite material, wherein the controller further is adapted to control the cooling element. Such a cooling element may be used to rapidly cool the zeolite material and to deflate the cuff as a result. This for example may be useful in use cases where a series of blood pressure measurements need to be performed over a relatively short period of time in which the interval between two successive blood pressure measurements is too short for the zeolite material to spontaneously cool to a target temperature at the start of a blood pressure measurement, e.g. a target temperature corresponding to a baseline pressure within the cuff. For example, the controller may be adapted to periodically heat and subsequently cool the zeolite material with the heating element and the cooling element respectively in order to perform a series of blood pressure measurements with the blood pressure measurement device. The heating element and the cooling element may be discrete (separate) elements or alternatively may be embodied by a single element capable of performing both heating and cooling functions.

In a second set of embodiments, the at least the first matter comprises a first electrolyte solution and a second electrolyte solution, and the chamber comprises an electrochemical reactor having a first cell comprising the first electrolyte solution and a second cell comprising the second electrolyte solution, and wherein the electrode arrangement comprises a first electrode in the first cell and a second electrode in the second cell to release said gas by an electrochemical reaction between the first electrolyte solution and the second electrolyte solution. In this set of embodiments, one or more gases are produced by the electrochemical decomposition of the first electrolyte and/or the second electrolyte solution, such as through water electrolysis by way of non-limiting example.

In the second set of embodiments, the controller may be adapted to deploy a direct current having a first polarity to the electrode arrangement to inflate the inflatable compartment; and deploy a direct current having a further polarity opposite to the first polarity to the electrode arrangement to deflate the inflatable compartment. In this manner, the blood pressure measurement device may be operated in a cyclical manner in which the gases generated with the direct current having the first polarity to inflate the cuff may be recombined by the deployment of the direct current having the further polarity to deflate the cuff. In some embodiments, the electrochemical reactor may be operated as a fuel cell when the direct current having the further polarity is deployed to the electrode arrangement and the energy generated by the recombination of the aforementioned gases may be used to recharge the power supply in the power supply unit, e.g. a rechargeable battery.

Alternatively, the inflatable compartment further comprises a pressure release valve under control of the controller such that any gas used to inflate the cuff can be released from the cuff upon completion of a blood pressure measurement to deflate the cuff, thereby providing a blood pressure measurement device that can be operated in a simple and straightforward manner.

In another embodiment, the inflatable compartment is fluidly coupled to the first cell, with the cuff further comprising a further inflatable compartment fluidly coupled to the second cell. Consequently, gases generated in the respective half reactions in both the first and second cells may be used to inflate the cuff, thereby increasing the efficiency of the cuff inflation and reducing its inflation time due to the fact that both the gases generated in the electrochemical reaction are being harvested and used for the inflation of the cuff.

### BRIEF DESCRIPTION OF THE DRAWINGS

Embodiments of the invention are described in more detail and by way of non-limiting examples with reference to the accompanying drawings, wherein:
FIG. 1 schematically depicts a blood pressure measurement device according to an embodiment;
FIG. 2 is a graph depicting zeolite gas storage capacity as a function of temperature;
FIG. 3 shows an enlarged part of the graph of FIG. 2;
FIG. 4 schematically depicts a blood pressure measurement device according to another embodiment;
FIG. 5 schematically depicts a blood pressure measurement device according to yet another embodiment;
FIG. 6 schematically depicts a blood pressure measurement device according to yet another embodiment in a deflated state;
FIG. 7 schematically depicts the blood pressure measurement device of FIG. 6 in an inflated state;
FIG. 8 schematically depicts a blood pressure measurement device according to a further embodiment;
FIG. 9 schematically depicts an example aspect of a blood pressure measurement device according to some of the herein described embodiments;
FIG. 10 schematically depicts a blood pressure measurement device according to still a further embodiment;
FIG. 11 schematically depicts a blood pressure measurement device according to still a further embodiment in a first mode of operation;
FIG. 12 schematically depicts the blood pressure measurement of FIG. 10 in a second mode of operation;
FIG. 13 schematically depicts a blood pressure measurement device according to yet a further embodiment;
FIG. 14 schematically depicts a blood pressure measurement device according to yet a further embodiment; and
FIG. 15 schematically depicts an aspect of a blood pressure measurement device according to an example embodiment.

### DETAILED DESCRIPTION OF THE EMBODIMENTS

It should be understood that the Figures are merely schematic and are not drawn to scale. It should also be understood that the same reference numerals are used throughout the Figures to indicate the same or similar parts.

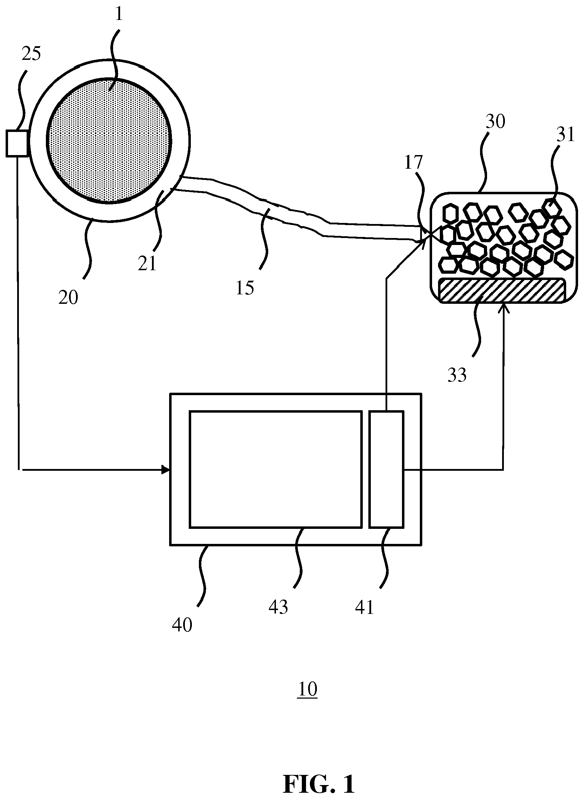

FIG. 1 schematically depicts a blood pressure measurement device 10 according to an embodiment of the present invention. The blood pressure measurement device 10 comprises an inflatable cuff 20 having an inflatable compartment 21 for enveloping a body portion 1 of a patient, such as for example an upper arm (e.g. bicep), wrist or finger. The inflatable compartment 21 may be delimited by any suitable material that allows for the inflation and deflation of the inflatable cuff 20, such as a suitable polymer material or the like. Such materials are well-known per se and are therefore not explained in further detail for the sake of brevity only.

The inflatable compartment 21 is fluidly connected to a chamber 30 through a length of tubing 15, e.g. tubing of a gas-tight material such as a gas-tight rubber or elastomer material. The chamber 30, which may be made of any suitable material, e.g. a polymer material, a metal or metal alloy, a composite material and so on, comprises a zeolite material 31 in thermal contact with an electrode arrangement 33 implementing a heating element, e.g. a resistive heating element such as a heating foil or a heating resistor. In the context of the present application, an electrode arrangement refers to any arrangement comprising one or more elements that can deploy an electromagnetic stimulus to the matter within the chamber 30, here the zeolite material 31. More specifically, the electrode arrangement is adapted to either deploy a current across such matter or alternatively to convert such a current into heat as is the case in the present embodiment.

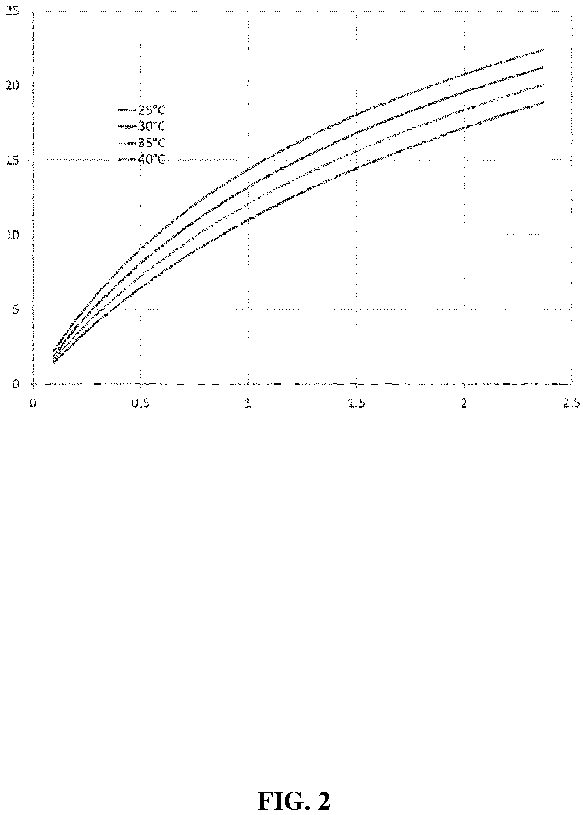

The zeolite material 31 may be provided in any suitable form such as in the form of beads. In accordance with the embodiments of the present invention, a gas is adsorbed to the surface of the zeolite material 31 prior to storage into the chamber 30. It is well-known per se that zeolites have a high surface area capable of adsorbing substantial amounts of gas. For example, a zeolite material 31 such as Nitroxy range of molecular sieves as marketed by the Arkema chemical company of Colombes, France, is capable of adsorbing nitrogen gas, amongst others. Importantly, the adsorption capacity of such zeolite materials is temperature-dependent, as is depicted in FIG. 2, which depicts the adsorbed amount of nitrogen in liters per liter zeolite of Arkema's Nitroxy Revolution zeolite as a function of temperature (°C) and pressure (bar). The different curves depict the different adsorption capacities of this zeolite at different temperatures.

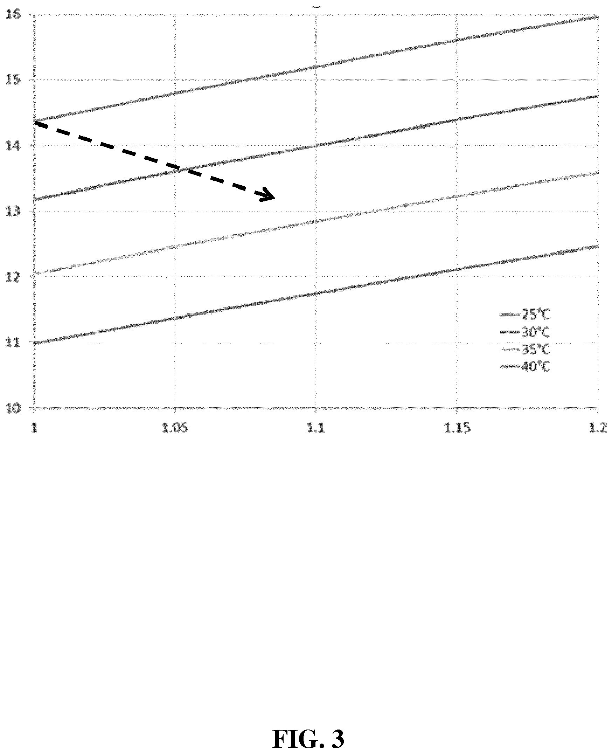

The adsorption characteristics of this zeolite around atmospheric pressure (∼ 1.1 bar) are magnified in FIG. 3. As highlighted by the dashed arrow, an increase in the temperature of the zeolite material 31 with the heating element implemented by the electrode arrangement 33 causes an increase in the pressure within the inflatable compartment 21 of the inflatable cuff 20 due to the release of adsorbed gas resulting from the decreased adsorption capacity of the zeolite material 31 at higher temperature. For example, for this particular zeolite material 31, an increase in temperature from 25°C to 35°C releases more than 1 L of gas per 1 L of zeolite material 31, which increases the pressure from 1.0 bar to 1.1 bar (∼ 80 mm Hg). Of course, other types of zeolites may be used within the chamber 30 as long as such zeolites have the desired temperature-dependent adsorption capacity of one or more target gases such that the cuff 20 can be inflated by releasing such one or more gases from the zeolite material 31 by heating the material.

The electrode arrangement 33 is conductively coupled to a power supply unit 40 including a controller 41 and a power supply 43. The power supply 43 may be a battery such as a non-rechargeable or a rechargeable battery. Alternatively, the power supply 43 may be omitted in case of a mains powered blood pressure measurement device 10 although this obviously is not preferred in embodiments in which the blood pressure measurement device 10 should be portable and/or wearable. The controller 41 typically is coupled between the power supply 43 and the electrode arrangement 33 and is adapted to deliver a current such as a DC current originating from the power supply 43 to the electrode arrangement 33 in order for the electrode arrangement 33 to heat the zeolite material 31 in the chamber 30.

The cuff 20 may further comprise a pressure sensor 25 that is communicatively coupled to the power supply unit 40 and to the controller 41 in particular, with the controller 41 being configured to generate the DC current in response to a pressure sensor reading provided by the pressure sensor 25. This may be a reading of the pressure within the inflatable compartment 21 or a reading of the pressure exerted by the cuff 20 onto the body portion 1 of the patient. This allows the controller 41 to accurately control the release of the gas from the zeolite material 31 by controlling the duration of the application of the DC current to the electrode arrangement 33. In particular, the controller 41 may terminate such a current upon receiving pressure information from the pressure sensor 25 indicative of the pressure sensed with the pressure sensor 25 having reached a target value, thereby accurately controlling the pressure within the inflatable compartment 21.

Alternatively or additionally, the blood pressure measurement device 10 may further comprise a valve 17 for regulating the gas flow from the chamber 30 to the inflatable chamber 21 of the cuff 20 under control of the controller 41 in order to control the pressure within the cuff 20. The valve 17 is shown as being located at an outlet of the chamber 30 by way of non-limiting example only; it should be understood that the valve 17 may be located in any suitable location, e.g. at an inlet of the inflatable compartment 21, in the length of tubing 15 or any other suitable location within the chamber 30. Although not specifically shown, it will be immediately understood by the skilled person that the blood pressure measurement device 10 typically comprises a user interface including a display or the like to allow a user of the blood pressure measurement device 10 to operate the device and to interpret blood pressure measurement readings received by the controller 41 from one or more pressure sensors such as the pressure sensor 25 within the cuff 20 and the displayed on the display device of the blood pressure measurement device 10. Such a user interface is entirely conventional and may take any suitable shape, and is therefore not explained in further detail for the sake of brevity only.

Furthermore, it should be understood that the blood pressure measurement device 10 may further comprise a data storage device for storing blood pressure measurement readings obtained by operation of the inflatable cuff 20 as explained above, for example to store a series of blood pressure measurements over a period of time for subsequent evaluation of such measurements. Such subsequent evaluation may take place using the user interface of the blood pressure measurement device 10 or alternatively the blood pressure measurement device 10 may comprise a communication module such as a wireless communication interface, a USB port or the like through which such stored readings may be extracted from the data storage device in the blood pressure measurement device 10 for evaluation on an external device such as a computer, tablet computer, smart phone, dedicated console and so on. Where the blood pressure measurement device 10 comprises such a communication module, the data storage device alternatively may be omitted from the blood pressure measurement device 10 in which case the communication module may be used to communicate an actual blood pressure reading to an external data storage device to facilitate subsequent evaluation of such a blood pressure reading or a sequence of such blood pressure readings as previously explained.

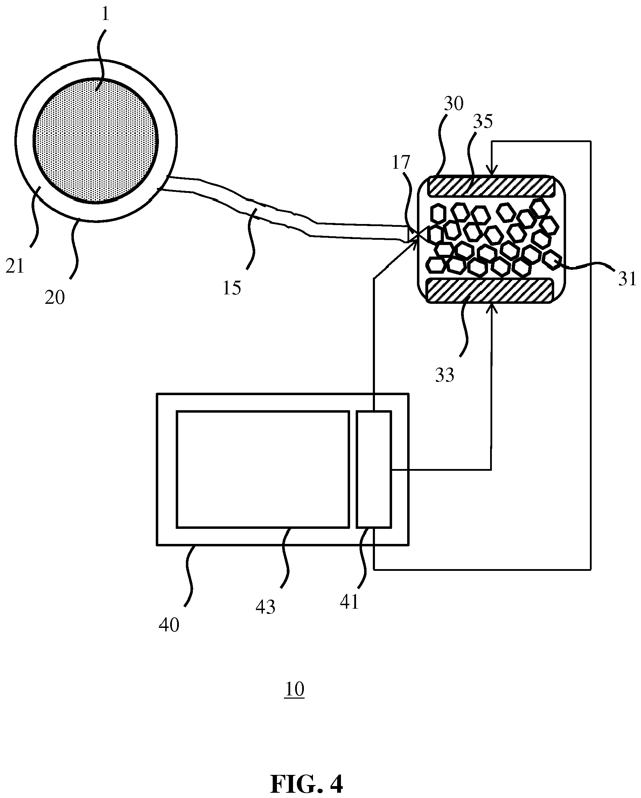

The zeolite-based blood pressure measurement device 10 typically is a closed system as the gas adsorption by the zeolite material 31 is reversible. More specifically, upon cooling of the zeolite material 31, the inflatable compartment 21 of the cuff 20 is deflated as the gas is re-adsorbed by the zeolite material 31 due to the increased adsorption capacity of the zeolite material 31 at lower temperatures. Such cooling of the zeolite material 31 may of the spontaneously, i.e. naturally through heat exchange between the zeolite material 31 and its surroundings. However, in an alternative embodiment as schematically depicted in FIG. 4, the blood pressure measurement device 10 further comprises a cooling element 35 thermally coupled to the zeolite material 31 under control of the controller 41 in order to increase the rate of cooling of the zeolite material 31 such that the inflatable compartment 21 of the cuff 20 is deflated more rapidly. Such a cooling element 35 may be an active cooling element such as a Peltier element or the like, or may be a passive cooling element such as a fan or the like. The cooling element 35 may be separate to the heating element 33 or alternatively a single element capable of both heating and cooling may be provided within the chamber 30 to heat and cool the zeolite material 31.

The presence of both heating and cooling capability within the chamber 30 enables periodic blood pressure measurements, i.e. semi-continuous measurements, with the blood pressure measurement device 10 in which the zeolite material 31 is first heated with the electrode arrangement implementing the heating element 33 to inflate the cuff 20 and subsequently rapidly cooled with the cooling element 35 to deflate the cuff 20. In this manner, a series of blood pressure measurements may be performed at a higher frequency than possible without the cooling element 35 due to the more rapid deflation of the inflatable chamber 21 of the cuff 20 by the more rapid cooling of the zeolite material 31 using the cooling element 35.

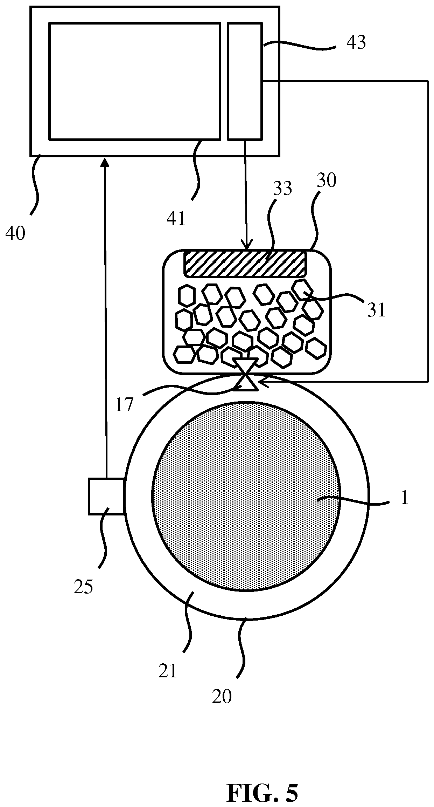

It is noted at this point that although the power supply unit 40 is shown as a separate entity to the chamber 30, it is of course equally feasible that the power supply unit 40 is integrated in a unit comprising both the chamber 30 and the power supply unit 40. Moreover, the chamber 30 does not need to be attached to the cuff 20 through a length of tubing 15. As schematically depicted in FIG. 5, the chamber 30 may be mounted on the cuff 20 with the valve 17 if present arranged between the chamber 30 and the inflatable compartment 21 of the cuff 20 such that the length of tubing 15 may be omitted. As previously mentioned, the chamber 30 may be mounted together with the power supply unit 40 onto the cuff 20, e.g. in a control unit or the like, in order to yield a blood pressure measurement device 10 that can be worn in its entirety on a body part 1 of a patient such as the patient's upper arm, wrist or finger, thus making the blood pressure measurement device 10 truly portable, even in a scenario where the power supply unit 40 is not mounted on the cuff 20, as in such a scenario the power supply unit 40 may be worn by the patient in a pocket of a garment or the like, with the power supply unit 40 being connected to the chamber 30 through one or more wires or cables.

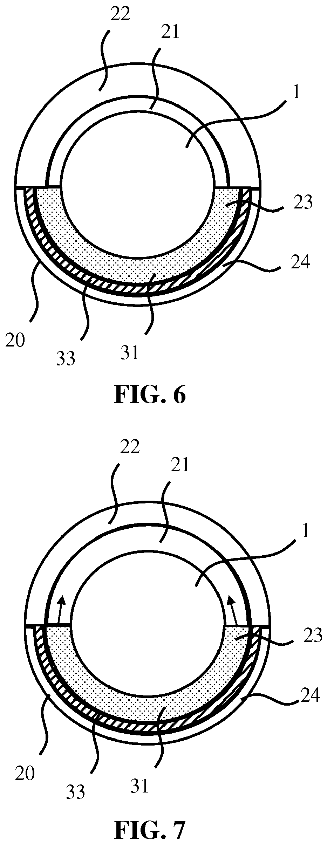

FIG. 6 schematically depicts yet another embodiment of the blood pressure measurement device 10, here implemented as a finger cuff in which the cuff 20 also houses the chamber 30 in a further compartment 23 containing the zeolite material 31. The electrode arrangement 33 in this embodiment may be implemented as a semi-cylindrical heating element in thermal contact with the zeolite material 31, with the electrode arrangement 33 being shielded from the outside world by an electrically and thermally insulating layer 24, e.g. a polymer layer of a suitable electrically and thermally insulating polymer. Alternatively, the layer 24 may be made of a heat conductive material or material having a high heat capacity in order to facilitate rapid cooling of the zeolite material 31, e.g. to facilitate highfrequency periodic blood pressure measurements as previously explained. Alternatively, although not explicitly shown, the finger cuff may further comprise a cooling element such as previously explained cooling element 35 in thermal contact with the zeolite material 31 to facilitate such rapid cooling.

The outer wall 22 surrounding the inflatable compartment 21 typically is made of a stiff, i.e. non-elastomeric, material such that inflation of the inflatable compartment 21 by release of the gas from the zeolite material 31 as indicated by the arrows in FIG. 7 causes a buildup of pressure on the body portion 1 of the patient. This is well-known per se and is therefore not explained in further detail for the sake of brevity only. The power supply unit 40 is not specifically shown in FIG. 6 and 7. It is noted that in this embodiment, the power supply unit 40 may be deployed in any suitable manner, e.g. as a separate unit that is connected through one or more wires or cables to the finger cuff to facilitate power and data communication between the finger cuff and the power supply unit. For example, the wearer of the finger cuff may wear the power supply unit 40 on his or her body, or in the pocket of a garment as previously explained.

In this embodiment, it is estimated that the required temperature change of the zeolite material 31 in order to sufficiently inflate the inflatable compartment 21 is about 5°C assuming that the volume of the inflatable compartment 21 is about 5 mm³. Of course, other dimensions of the inflatable compartment 21 may be contemplated, in which case the required temperature change of the zeolite material 31 may be adjusted accordingly.

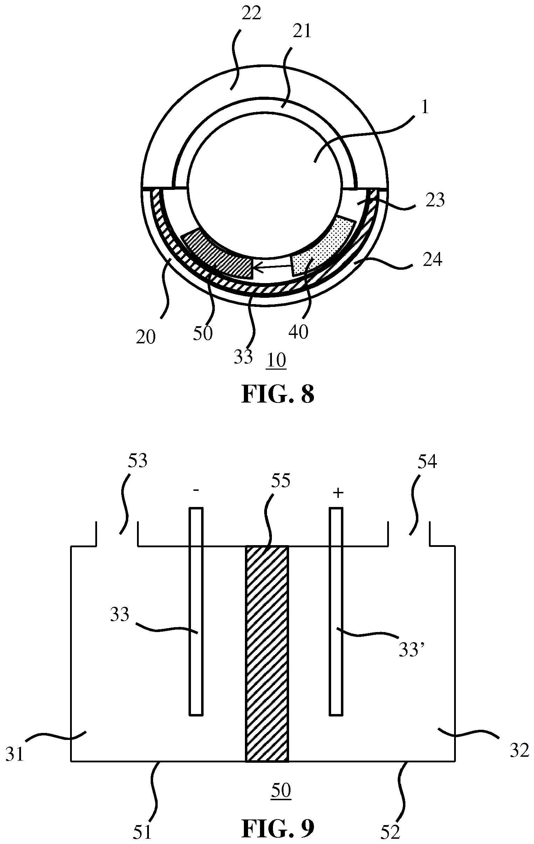

FIG. 8 schematically depicts a blood pressure measurement device 10 according to another set of embodiments, here depicted as a finger cuff by way of non-limiting example only as the blood pressure measurement device 10 according to this set of embodiments may be implemented in any of the previously described arrangements. In this set of embodiments, the zeolite material 31 is replaced by an electrochemical reactor 50. A typical electrochemical reactor 50 is schematically depicted in FIG. 9. The electrochemical reactor 50 typically comprises a first cell 51 including a first electrolyte solution 31 and a second cell 52 including a second electrolyte solution 32, with the first cell 51 being separated from the second cell 52 by a separator 55, e.g. a diaphragm, membrane or the like. The separator 55 may be semipermeable, e.g. to allow ion transport between the first cell 51 and the second cell 52. By way of non-limiting example, the separator 55 may be an ionomer membrane such as a sulfonated tetrafluoroethylene based fluoropolymer-copolymer or any other suitable ionomer membrane.

The first cell 51 typically comprises a first electrode 33 such as an anode of the electrode arrangement whereas the second cell 52 typically comprises a second electrode 33' such as a cathode of the electrode arrangement, with the first electrode 33 and the second electrode 33' being coupled to the controller 41 of the power supply unit 50 such that upon application of a current such as a DC current across the electrode arrangement, an electrochemical reaction (e.g. a redox reaction) is induced in the electrochemical reactor 50 causing the release of a first gas from the first electrolyte solution 31 in the first cell 51 and optionally the release of a second gas from the second electrolyte solution 32 in the second cell 52. The first electrode 33 and the second electrode 33'may comprise any suitable electrode material including but not being limited to platinum, silver, titanium and copper.

The first cell 51 may comprise a first gas outlet 53 through which the first gas can escape the first cell 51, whilst the second cell 52 may comprise a second gas outlet 54 through which the second gas can escape the second cell 52. Alternatively, the second gas may be bound to a metal ion in the second electrolyte solution 32, e.g. a Fe³⁺ ion in case of an electrolyte solution comprising a dissolved salt such as Fe₂(SO₄)₃, in which case the second gas outlet 54 may be omitted. At least one of the first gas outlet 53 and the second guess outlet 54 may be fluidly coupled to the inflatable compartment 21 of the cuff 20 such that the cuff 20 can be inflated by a gas generated in the electrochemical reaction (i.e. the electrochemical decomposition) of the first electrolyte solution 31 and/or the second electrolyte solution 32 as will be explained in further detail below. Any suitable electrolyte solutions may be used for the first electrolyte solution 31 and the second electrolyte solution 32. In an example embodiment, the electrolyte solutions are water-based in which case the electrochemical reaction is water electrolysis during which hydrogen gas is generated at the cathode and oxygen gas is generated at the anode of the electrochemical reactor 50.

The controller 41 may be operable to provide the electrochemical reactor 50 with a DC current across the electrode arrangement 33, 33' in a first mode of operation in which the electrochemical reaction produces the one or more gases as explained above to inflate the inflatable compartment 21 of the cuff 20. The cuff 20 for example may be operated to be kept at a baseline pressure, with the electrochemical reaction being used to temporarily increase the pressure within the cuff 20 to facilitate a blood pressure measurement with the blood pressure measurement device 10. The baseline pressure may be about atmospheric pressure for example. The initial baseline pressure within the inflatable compartment 21 may be generated with the electrochemical cell 50 or alternatively a small air pump (not shown) may be included in the design of the blood pressure measurement device 10 in order to more rapidly bring the inflatable compartment 21 to the baseline pressure, after which the multiple blood pressure measurements may be performed silently by altering the baseline pressure with the electrochemical reactor 50.

After the measurement, the cuff 20 may be returned to its baseline pressure by removing excess gas from the inflatable compartment 21, e.g. by venting the excess gas into ambient through a valve fluidly coupling the inflatable compartment 21 to the ambient, or alternatively by operating the electrochemical reactor 50 in a fuel cell mode, e.g. when the gas used to inflate the inflatable compartment 21 is hydrogen gas, in which case the hydrogen gas in the inflatable compartment 21 may be recombined with oxygen gas to form water.

The energy generated in such a fuel cell mode may be used to recharge a rechargeable battery used as the power supply 43, thereby prolonging the lifetime of the power supply 43. Alternatively, the controller 41 may be operable in a second mode of operation in which a direct current of opposite polarity to the direct current used in the first mode of operation is applied across the electrode arrangement 33, 33' to invoke the reversal of the electrochemical reaction deployed in the first mode of operation, i.e. to at least partially deflate the inflatable compartment 21. As such fuel cell or energy recovery architectures are well-known per se, they are not explained in further detail for the sake of brevity only. It suffices to say that any suitable architecture design may be used for this purpose.

The feasibility of a blood pressure measurement device 10 based on such an electrochemical reactor 50 can be readily understood from the following. Assuming a volume of 1 cm³ of the inflatable compartment 21, in order to perform a two-minute blood pressure measurement every 20 minutes over 24 hour period, 30 cm³ of gas needs to be generated by the electrochemical reactor 50. In comparison, 1 cm³ of water can generate 600 cm³ of molecular hydrogen gas such that an electrochemical reactor 50 occupying the same volume as the inflatable compartment 21 can facilitate continuous use of such a blood pressure measurement device 10 for up to 20 days. Moreover, in order to bring the pressure in the cuff 20 to 1.1 bar in 15 seconds, which is a generally acceptable initiation time for a blood pressure measurement device, about 85 mA is required using electrodes having a surface area of about 1 cm², which is small enough to fit within a finger cuff as schematically depicted in FIG. 8. The power supply 43 also can be kept small enough to be integrated within such a finger cuff as such a battery needs to provide about 0.1 Wh of energy at an operating voltage of 2V, which may be provided for example by a lithium ion battery having a weight of less than 1 g, i.e. a volume of less than 1 cm³.

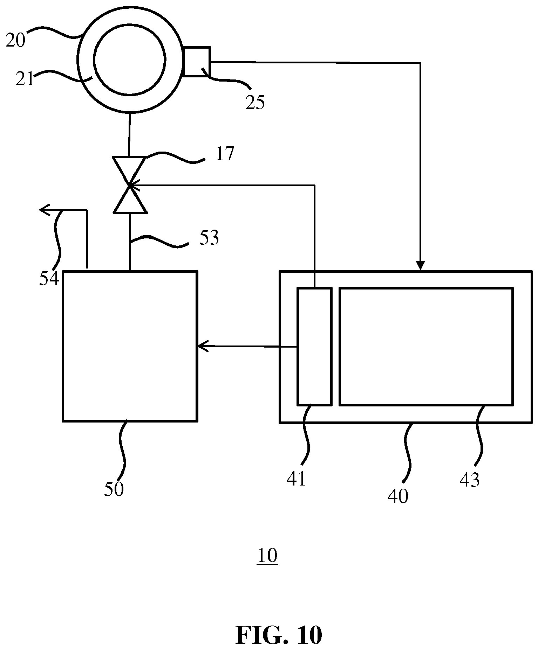

A first example embodiment of a blood pressure measurement device 10 comprising such an electrochemical reactor 50 is schematically depicted in FIG. 10. Here the first gas outlet 53, e.g. an outlet for hydrogen gas or any other suitable gas to be generated during the electrochemical reactor 50, is fluidly coupled to the inflatable compartment 21 of the cuff 20 through an optional valve 17 under control of the controller 41. During operation in a first mode, the controller 41 deploys a DC current across electrodes 33, 33' of the electrochemical reactor 50 to inflate the cuff 20 to a desired pressure, e.g. as measured with a pressure sensor 25 as previously explained, with the gas generated in the first cell 51 of the electrochemical reactor 50. In this embodiment, the gas generated in the second cell 52 of the electrochemical reactor 50 is vented through the second gas outlet 54 to ambient, e.g. the gas may be oxygen gas in case of water electrolysis in the electrochemical reactor 50 as previously mentioned. Alternatively, the oxygen gas may be dissolved in the second electrolyte solution 32 as previously explained. In this embodiment, the first cell 51 of the electrochemical reactor 50 forms a closed system with the inflatable compartment 21 of the cuff 20 such that any hydrogen gas generated in the first cell 51 to inflate the cuff 20 may be recombined with oxygen retrieved from ambient or from the second electrolyte solution 32 in a reverse reaction as previously explained.

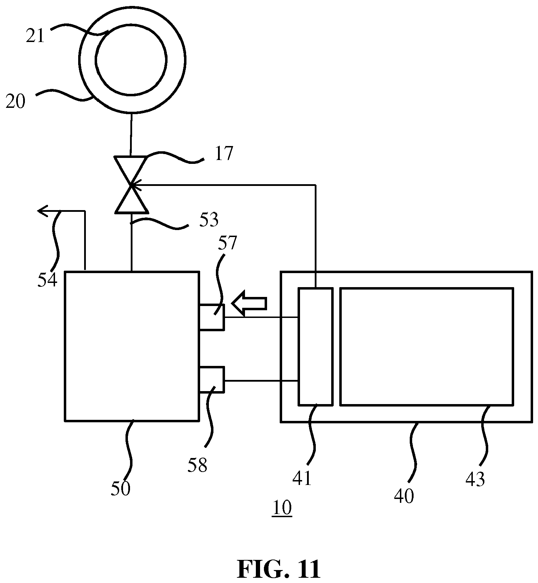

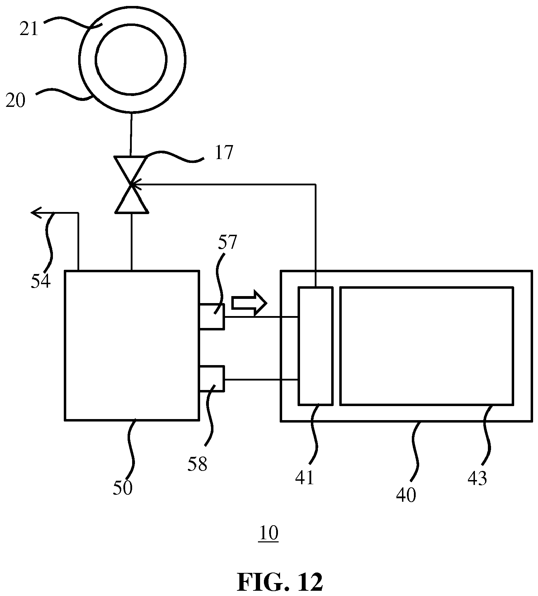

This is schematically shown in FIG. 11 and FIG. 12. In FIG. 11 the blood pressure measurement device 10 is operated in a first mode of operation in which the controller 41 deploys a direct current obtained from the power supply 43 in the power supply unit 40 across the electrode arrangement 33, 33' of the electrochemical reactor 50, here symbolized by terminals 57, 58 on the electrochemical reactor 50, to induce the electrochemical reaction generating the gas for inflating the cuff 20, as indicated by the block arrow pointing towards the terminal 57.

In the second mode of operation, as depicted in FIG. 12 by the block arrow pointing away from the terminal 57 of the electrochemical reactor 50, a reverse electrochemical reaction is induced in the electrochemical reactor 50 in which hydrogen gas is extracted from the inflatable compartment 21 of the cuff 20 and recombined with oxygen retrieved from ambient or from the second electrolyte solution 32 to reform water. This may be achieved by the provision of a direct current of opposite polarity across the electrode arrangement 33, 33' with the controller 41 or by operating the electrochemical reactor 50 in the fuel cell mode as previously explained. The direction of flow of hydrogen gas may be controlled with the optional valve 17 under control of the controller 41, e.g. to maintain pressure within the cuff 20 during a blood pressure measurement and to facilitate deflation of the cuff 20 after such a measurement. In this manner, a blood pressure measurement device 10 may be provided in which pressure modulations in the cuff 20 can be realized in a straightforward manner by the deployment of direct currents having different polarities to the electrode arrangement 33, 33' of the electrochemical reactor 50 with the controller 41.

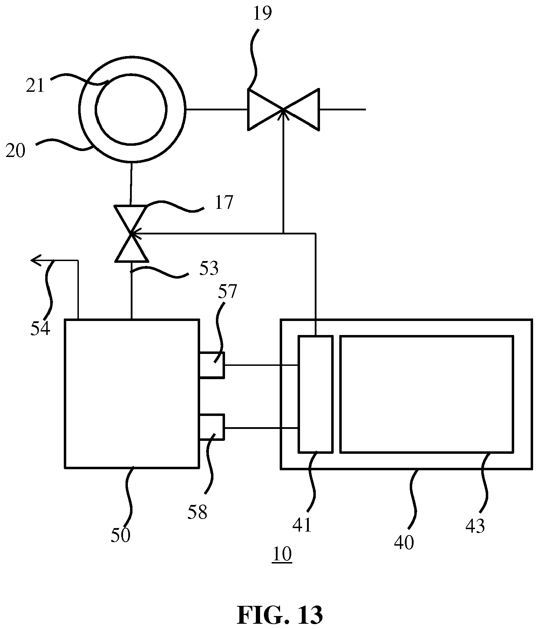

FIG. 13 schematically depicts an alternative embodiment of such a blood pressure measurement device 10. In this embodiment, the deflation of the inflatable compartment 21 of the cuff 20 is realized through a further valve 19 connecting the inflatable compartment 21 to ambient under control of the controller 41 such that upon completion of a blood pressure measurement, the controller 41 may control the further valve 19 to deflate the inflatable compartment 21 of the cuff 20, e.g. to a baseline pressure as previously explained. Although not shown in FIG. 13, the controller 41 may control the further valve 19 as a function of a cuff pressure measured with the pressure sensor 25 to accurately control the pressure in the cuff 20 as previously explained. Hence, in this embodiment, the gas generated in the electrochemical reaction to inflate the inflatable compartment 21 of the cuff 20 is not recycled but rather released to ambient. Hence, an electrolyte solution composition may be chosen that generated a relatively harmless gas for the inflation of the cuff 20, as will be readily understood by the skilled person.

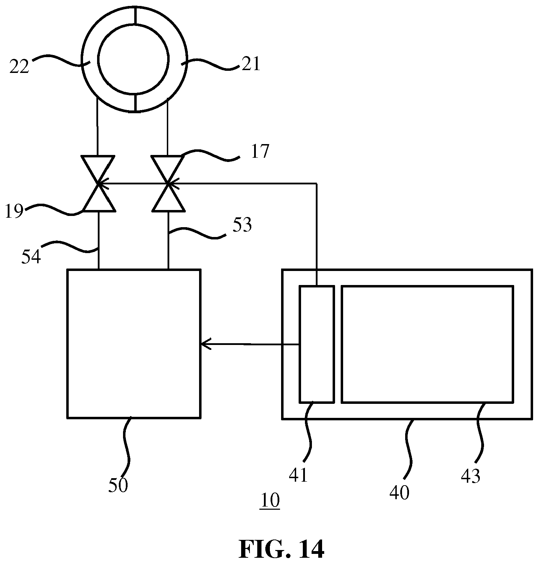

FIG. 14 schematically depicts another alternative embodiment of such a blood pressure measurement device 10. In this embodiment, the cuff 20 comprises a further inflatable compartment 22 in addition to the inflatable compartment 21. The further inflatable compartment 22 to the gas outlet 54 of the second cell 52 of the electrochemical reactor 50 such that in case of both cells 51, 52 producing a gas as a reaction product of the electrochemical reaction in the electrochemical reactor 50, both gases may be harvested to inflate the cuff 20, such that the cuff 20 may be more rapidly inflated compared to a cuff 20 having a single inflatable compartment 21 inflated by only one of the two gases that are released as reaction products of such an electrochemical reaction.

Valves 17 and 19 may be present in the fluidic connections between the first cell 51 and the first inflatable compartment 21 and between the second cell 52 and the further inflatable compartment 22 respectively. The valves 17 and 19 may be individually controlled by the controller 41, e.g. in response to pressure sensor data provided by one or more pressure sensors (not shown) on the cuff 20 to accurately control the pressure in the cuff 20. The gases may be recombined when deflating the cuff 20 as previously explained, or each of the inflatable compartments 21, 22 may be fluidly coupled to a release valve (not shown) under control of the controller 41 to vent the gases to ambient when deflating the cuff 20 as previously explained.

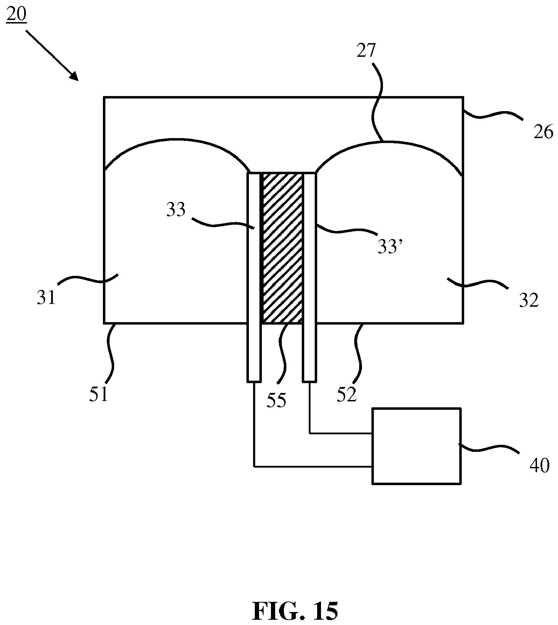

FIG. 15 schematically depicts an aspect of the blood pressure measurement device 10 including such an electrochemical reactor 50 within the cuff 20. In this embodiment, the cuff 20 has a flexible inner wall 27 in contact with the electrolyte solutions 31, 32 in the electrochemical reactor 50 and a rigid outer wall 26 such that upon generation of the gases in the cells 51, 52 of the electrochemical reactor 50, the flexible inner wall 27 is compressed by the gases to increase the pressure within the cuff 20.

It should be noted that the above-mentioned embodiments illustrate rather than limit the invention, and that those skilled in the art will be able to design many alternative embodiments without departing from the scope of the appended claims. In the claims, any reference signs placed between parentheses shall not be construed as limiting the claim. The word "comprising" does not exclude the presence of elements or steps other than those listed in a claim. The word "a" or "an" preceding an element does not exclude the presence of a plurality of such elements. The invention can be implemented by means of hardware comprising several distinct elements. In the device claim enumerating several means, several of these means can be embodied by one and the same item of hardware. The mere fact that certain measures are recited in mutually different dependent claims does not indicate that a combination of these measures cannot be used to advantage.

## Claims

1. A blood pressure measurement device (10) comprising:
a cuff (20) having an inflatable compartment (21) in fluid communication with a chamber (30) comprising an electrode arrangement (33, 33') contacting at least a first matter (31, 32) capable of releasing a gas having a composition different to said matter for inflating the inflatable compartment in response to an electromagnetic stimulus applied to the at least the first matter by the electrode arrangement; and
a power supply unit (40) including a controller (41) communicatively coupled to the electrode arrangement adapted to control the power supply unit to provide said electromagnetic stimulus to the electrode arrangement.

2. The blood pressure measurement device (10) of claim 1, further comprising a pressure sensor (25) in said cuff communicatively coupled to the controller (41), wherein the controller is adapted to control the power supply unit (40) to provide said electromagnetic stimulus to the electrode arrangement (33, 33') as a function of a pressure in the inflatable compartment (21) sensed by the pressure sensor.

3. The blood pressure measurement device (10) of claim 1 or 2, further comprising a valve (17, 19) in fluid communication with the inflatable compartment (21), wherein the controller (41) is arranged to control said valve to regulate inflation and/or deflation of the inflatable compartment.

4. The blood pressure measurement device (10) of any of claims 1-3, wherein the chamber (30) and the power supply unit (40) are housed in a control unit in fluid communication with the inflatable compartment (21) through a length of tubing (15).

5. The blood pressure measurement device (10) of any of claims 1-3, wherein the chamber (30) is housed in a control unit mounted on the cuff (20).

6. The blood pressure measurement device (10) of any of claims 1-3, wherein the cuff (20) comprises a further compartment (22) housing the chamber (30) in fluid communication with the inflatable compartment (21).

7. The blood pressure measurement (10) device of claim 6, wherein the cuff (20) is dimensioned to fit around a finger.

8. The blood pressure measurement device (10) of any of claims 1-7, wherein the first matter (31) comprises a zeolite material and wherein the electrode arrangement (33) comprises a heating element adapted to heat the zeolite material.

9. The blood pressure measurement device (10) of claim 8, further comprising a cooling element (35) in thermal contact with the zeolite material, wherein the controller (41) further is adapted to control the cooling element.

10. The blood pressure measurement device (10) of claim 9, wherein the controller (41) is adapted to periodically heat and subsequently cool the zeolite material with the heating element (33) and the cooling element (35) respectively.

11. The blood pressure measurement device (10) of any of claims 1-7, wherein the at least the first matter comprises a first electrolyte solution (31) and a second electrolyte solution (32), and the chamber (30) comprises an electrochemical reactor (50) having a first cell (51) comprising the first electrolyte solution and a second cell (52) comprising the second electrolyte solution, and wherein the electrode arrangement comprises a first electrode (33) in the first cell and a second electrode (33') in the second cell to release said gas by an electrochemical reaction between the first electrolyte solution and the second electrolyte solution.

12. The blood pressure measurement device (10) of claim 11, wherein the controller (41) is adapted to:
deploy a direct current having a first polarity to the electrode arrangement (33, 33') to inflate the inflatable compartment (21); and
deploy a direct current having a further polarity opposite to the first polarity to the electrode arrangement to deflate the inflatable compartment.

13. The blood pressure measurement device (10) of claim 11, wherein the inflatable compartment (21) further comprises a pressure release valve (19) under control of the controller (41).

14. The blood pressure measurement device (10) of any of claims 11-13, wherein the inflatable compartment (21) is fluidly coupled to the first cell (51), the cuff (20) further comprising a further inflatable compartment (22) fluidly coupled to the second cell (52).

15. The blood pressure measurement device (10) of any of claims 11-14, wherein the power supply unit (40) comprises a rechargeable battery (43) and the controller (41) is adapted to recharge said rechargeable battery with a current generated by a reversal of said electrochemical reaction.
